(19) Europäisches Patentamt
European Patent Office
Office européen des brevets

(11) Publication number: **0 169 937**
**A2**

(12) **EUROPEAN PATENT APPLICATION**

(21) Application number: 84114741.6

(22) Date of filing: 04.12.84

(51) Int. Cl.[4]: **C 07 K 5/06**
C 07 K 1/06, C 07 K 1/12

(30) Priority: 01.08.84 IT 2217784

(43) Date of publication of application:
05.02.86 Bulletin 86/6

(84) Designated Contracting States:
AT BE CH DE FR GB IT LI LU NL SE

(71) Applicant: FARCHEMIA S.P.A.
Via S. Giovanni Bosco 3
Treviglio (Bergamo)(IT)

(72) Inventor: Finotto, Martino
Via S. Giovanni Bosco, 3
Treviglio, (Bergamo)(IT)

(74) Representative: Bianchetti, Giuseppe
Studio Consulenza Brevettuale Via Senato 24
I-20121 Milan(IT)

(54) **Process for the preparation of alpha-L-aspartyl-L-phenylalanine alkyl esters.**

(57) A process for the preparation of α-L-aspartyl-L-phenylalanine alkyl esters, and namely of the methyl ester or aspartame, characterized by using, as starting material, a L-asparagine derivative having the amino group protected, which is reacted with an alkyl ester of L-phenylalanine to produce a dipeptide whose amido group is subsequently converted to carboxylic group by means of nitrite ions.

EP 0 169 937 A2

Croydon Printing Company Ltd

"Process for the preparation of α-L-aspartyl-L-phenylalanine alkyl esters"

The present invention relates to a new process for preparing α-L-aspartyl-L-phenylalanine alkyl esters of formula I

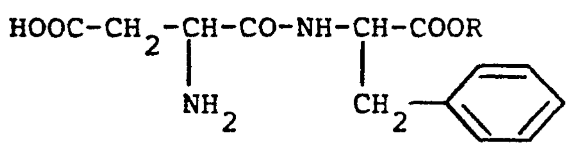

(I)

wherein R represents an alkyl group having from 1 to 5 carbon atoms.

In a particularly preferred aspect, the present invention provides a process for preparing α-L-aspartyl-L-phenylalanine methyl ester (I, wherein R = $CH_3$) or aspartame.

Aspartame is a well known sweetening agent widely used as dietetic sweetening agent, due to its taste very similar to that of saccharose.

Dipeptides of formula (I) are known to be prepared starting from L-aspartic acid derivatives. According to a known method, the process is carried out starting from L-aspartic acid in which both the $-NH_2$ and ß- and α-carboxylic groups have been protected by means, for example, of benzyloxycarbonyl or formyl or 4-nitrophenylester groups: by reaction with a L-phenylalanine ester the 4-nitrophenol is removed and the protected dipeptide is obtained, from which the other protecting groups can be removed by such conventional techniques as hydrogenolysis.

However, selective esterification of the ß-carboxylic group of L-aspartic acid is difficult to perform and, moreover, it takes place in low yields; on the other hand to produce aspartame it is necessary that condensation should occur only on the α-carboxylic group.

According to another known method a N-protected L-aspartic anhydride is employed as the starting material; however condensation of said anhydride with aminoacids or aminoacid esters (namely, L-phenylalanine methyl ester) followed by removal of the protecting group, always gives α and ß isomers in admixture which must be subsequently separated.

The drawbacks of the methods according to the prior art are overcome by the process of the present invention, which allows to obtain easily and in high yields α-L-aspartyl-L-phenylalanine alkyl esters, by means of a condensation occurring exclusively at position α.

The main advantage of the process according to the invention is that, instead of L-aspartic acid derivatives, there are employed as starting products L-asparagine derivatives, i.e. derivatives from a commercially available natural product having the ß-carboxylic group already "blocked".

According to the invention, a L-asparagine derivative of formula (II), in which the amino group has been protected, is condensed with a L-phenylala

nine alkyl ester (III) to give a dipeptide derivative (IV) the amido group of which is selectively transformed into a carboxylic group, the final step of deprotection of the amino group taking place by isolating or not the resulting intermediate (V), as shown in the following reaction scheme:

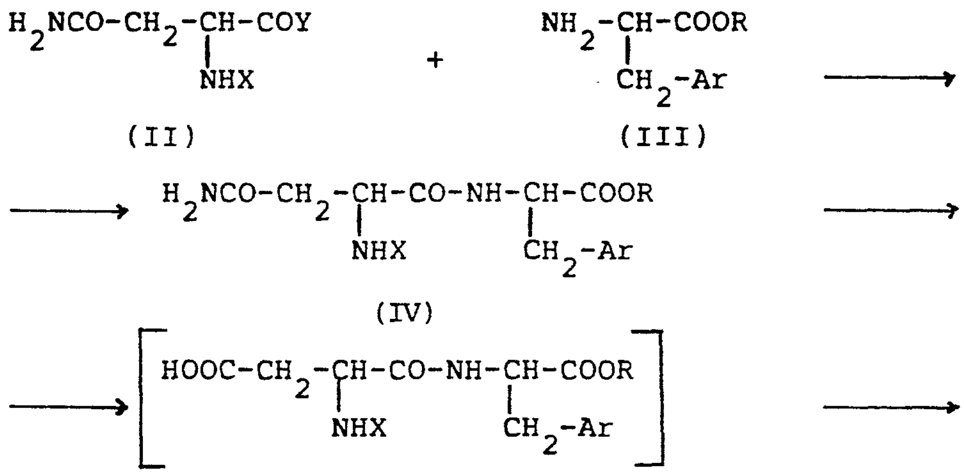

(II) (III)

(IV)

(V)

in which:

X represents a group protecting the amino moiety;

Y represents OH or an activating atom or group;

R has the above defined meanings; and

Ar represents a phenyl group.

More particularly, X represents α-haloacyl, formyl, benzyloxycarbonyl or trimethylsilyl groups; Y represents hydroxy, alcoxycarbonyloxy, o-nitrophenoxy or O-N-succinimido groups. Both X and Y can be inserted in the L-asparagine molecule easily and in high yields, the resulting derivatives being generally crystalline compounds which can be easily isolated in a pure form.

The condensation of the L-asparagine derivative (II) with a L-phenylalanine alkyl ester (III) is conveniently carried out under different conditions, depending on the nature of the Y group.

When Y = OH, the condensation with compound (III) may be advantageously carried out with dicyclohexylcarbodiimide in the presence of N-hydroxy-succinimide, in reaction mediums such as dimethylformamide. Alternatively, the same condensation may also be effected using pivaloyl chloride in the presence of tertiary bases, such as pyridine and N-ethylpiperidine, in solvents such as methylene chloride or dimethylformamide. On the other hand, when Y = o-nitrophenyl, the condensation may be carried out by simple treatment with compound (III) at room temperature, for example in acetonitrile/pyridine as the solvent.

The reaction mixture is concentrated under vacuum, then diluted with water and the resulting dipeptide (IV) is recovered by filtration. The dipeptide may be subsequently washed with a sodium carbonate solution, with diluted hydrochloric acid and with water and it may be used as such for the next step or it can be optionally recrystallized with, for example, methanol.

The conversion of asparaginyl-dipeptide (IV) in aspartyl-dipeptide (V) is carried out with nitrosonium salts, such as nitrosyl tetrafluoborate or sulfate, or with inorganic or organic nitrites in the presence of mineral acids.

The reaction is advantageously carried out by treating compound (IV) with nitrosylsulfuric acid in glacial acetic acid, or in acetonitrile or dimethylformamide, at room temperature for about 4 hours, under strong stirring. Compound (V) may be precipitated by addition of ice-water and recovered by filtration.

Alternatively, from the intermediates (IV) the L-asparaginyl-L-phenylalanine esters having formula (IVa)

$$H_2NOC-CH_2-\underset{NH_2}{\underset{|}{CH}}-CO-NH-\underset{CH_2-Ar}{\underset{|}{CH}}-COOR \qquad (IVa)$$

(wherein R and Ar have the above defined meanings) may be obtained.

Such esters may be, on their turn, protected at the amino group with alternative X groups, constituting the reference - mainly from the optical purity point of view - for the reaction products (II) + (III).

The final compound (I) is obtained from the intermediate (V) by removing the protecting group X from the nitrogen atom of the amino group, according to standard procedures. Thus, for X = benzyloxycarbonyl, an hydrogenolysis with palladium/charcoal (5-10%) is carried out, for example in methanol/water or acetic acid/water solution. If, on the contrary, X is trimethylsilyl or formyl, the deprotection may be carried out by heating in acid medium. In this case, when the transformation of the $-CONH_2$ group into carboxylic group is over, and optionally after destroying of the excess-$NO_2^-$ ion,

the elimination of the formyl or trimethylsilyl group without isolating the intermediate (V), can be directly carried out.

According to a preferred embodiment of the invention, X represents an α-haloacyl residue (such as, for example, chloroacetyl, α-bromo- or α-chloropropionyl, α-bromo- or α-chlorohexanoyl and the like). In this case, after the transformation of the $-CONH_2$ group into carboxyl group, the elimination of the α-haloacyl group is carried out by treatment with thiourea and decomposition of the resulting isothiouronium salt, according to the scheme:

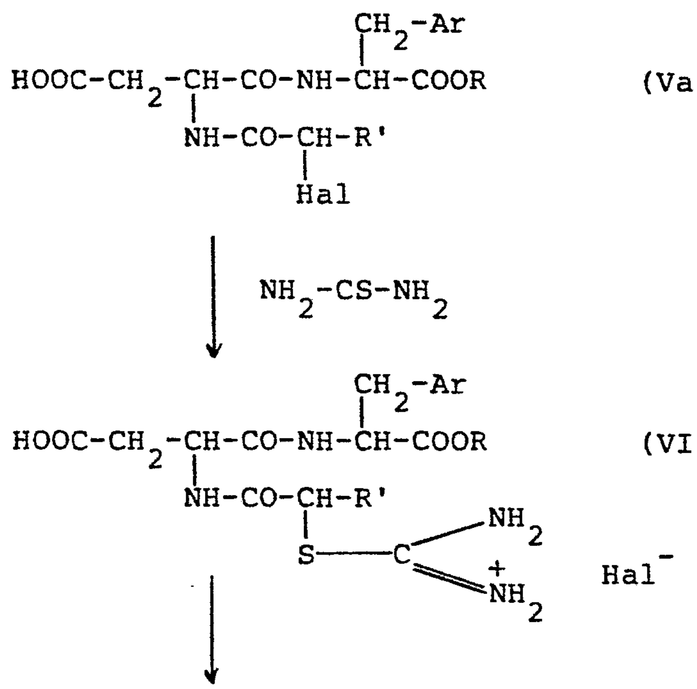


(Va)

(VI)

(I)

wherein R and Ar have the above defined meanings, Hal is an halogen atom (preferably chlorine or bromine), while R' is hydrogen or $C_1$-$C_5$ linear or branched alkyl residue.

The recovery of the isothiouronium salt (VI) is not necessary since the preparation of the final esters (I) from the haloacyl derivatives (Va), turns out to be very easy without isolating said salts, by direct treatment with water at pH 7-8, at temperatures ranging from 20 to 60°C, preferably about 40°C.

Anyway particularly pure esters (I) are obtained in very good total yields. Namely, by operating according to the invention, aspartame (I, R = $CH_3$) free from the beta form is obtained, with an average yield of 60-75% on the starting asparagine.

The following non limitative examples are reported for sake of illustration of the process according to the present invention.

EXAMPLE 1

a) Benzyloxycarbonyl-α-L-asparaginyl-L-phenylalanine (IV, wherein R = $CH_3$ and X = $-CO-O-CH_2-C_6H_5$)

77.5 Grams of benzyloxycarbonyl-L-asparagine o-nitrophenylester (II, wherein X = $CO-O-CH_2-C_6H_5$ and Y = 2-nitrophenoxy) was added at room temperature to a solution containing 65 g of L-phenylalanine methyl ester hydrochloride in 2 liters of a mixture 1/1 acetonitrile/pyridine. The reaction mixture was stirred for 8 hours, then concentrated under vacuum to half volume, diluted with water (3 liters) and stirred for 2 hours.

The resulting product was recovered by filtration and washed with a 5% sodium carbonate solution, with 2% hydrochloric acid, then with water.

After drying under vacuum at 50°C, 75 g of compound were obtained. Yield: 88%.

M.p. 195-197°C (methanol).

$[\alpha]_D^{25}$ + 16.5 (C = 2%, glacial acetic acid).

Elemental analysis:

For $C_{22}H_{25}N_3O_6$ (m.w. = 427.46)

Calcd.% C = 61.82; H = 5.89; N = 9.83

Found % C = 61.59; H = 6.01; N = 9.73.

b) Benzyloxycarbonyl-α-L-aspartyl-L-phenylalanine methyl ester (V, wherein R = $CH_3$ and X = $-CO-O-CH_2-C_6H_5$)

24 Grams of nitrosylsulfuric acid suspended in 100 ml of glacial acetic acid were added, during 30 minutes, to 42.7 g of the ester obtained in step a) suspended in 200 ml of glacial acetic acid. A strong nitrogen evolution took place, after which the solution was stirred for 3 hours at room temperature. After cooling to 0°C, 100 ml of water was added to destroy the excess nitrosonium salt and the compound was precipitated by addition of 2 liters of ice-water mixture, under stirring. After filtration, washing with water and drying under vacuum 35 g of the raw product (82% yield) was obtained, which was directly used for the next step.

c) α-L-Aspartyl-L-phenylalanine methyl ester (aspartame) (I, wherein R = $CH_3$)

30 Grams of the intermediate obtained in step b) was dissolved in 600 ml of a mixture methanol/water 70/30 (v/v). After addition of 4 g of 10% Pd/C catalyst, the reaction mixture was hydrogenated under

stirring for 4 hours. Thereafter the catalyst was removed by filtration, and the solution was concentrated under vacuum to 200 ml. The resulting product was separated by filtration and crystallized from water.

18 Grams of the product was obtained (87% yield); m.p. 246-247°C, $[\alpha]_D^{25}$ + 15.5° (C = 4%, formic acid 15N).

The chromatographic analysis showed that the product was pure α-L-aspartyl-L-phenylalanine methyl ester (aspartame); the present 3-L-benzyl-6-L-(carboxymethyl)-2,5-dioxopiperazine (aspartame diketopiperazine) was less than 0.5% (by HPLC).

EXAMPLE 2

Benzyloxycarbonyl-α-L-asparaginyl-L-phenylalanine methyl ester (IV, wherein R = $CH_3$ and X = $-CO-O-CH_2-C_6H_5$)

28.1 Ml of triethylamine was added under stirring to 43 g of L-phenylalanine hydrochloride methyl ester in 200 ml of DMF, cooled at 0°C. The reaction mixture was filtered and 53.3 g of benzyloxycarbonyl-L-asparagine and 23 g of N-hydroxy-succinimide were added to the solution. The mixture was cooled to 0°C and 41.3 g of dicyclohexylcarbodiimide dissolved in 150 ml of dimethylformamide was added. The reaction was continued for 4-6 hours at 5-10°C, then 10 ml of glacial acetic acid was added to destroy the non reacted dicyclohexylcarbodiimide, thereafter the mixture was kept under stirring at room temperature for 1 hour more,

filtered and diluted with water (2 liters).

The resulting precipitate was recovered by filtration, washed with a 5% sodium carbonate solution, with 2% hydrochloric acid, then with water. After drying under vacuum at 50°C, 70 g of compound was obtained, which was identical to that obtained in Example 1, step a). The obtained compound was transformed into aspartame according to the procedure described in steps b) and c) of Example 1.

EXAMPLE 3

α-L-Asparaginyl-L-phenylalanine methyl ester

(IVa, wherein R = $CH_3$)

42.7 Grams (0.1 moles) of benzyloxycarbonyl-α-L-asparaginyl-L-phenylalanine methyl ester was dissolved in 400 ml of dimethylformamide, thereafter 5 g of 10% Pd/C catalyst was added, and the mixture was hydrogenated under stirring for 4-6 hours at room temperature, checking the end of hydrogenolysis by chromatography. The catalyst was removed by filtration and the solution was concentrated to dryness; the resulting residue was taken up in methanol and precipitated again by addition of ethyl ether. The compound was separated by filtration, washed with ethyl ether and dried under vacuum; 27 g of compound was obtained (92% yield). $[\alpha]_D^{20°}$ +7.9° (c = 1, methanol). After acid hydrolysis of the peptide in closed vials for 22 hours in 6M HCl at 100°C, the aminoacid analysis showed a phenylalanine/aspartic acid ratio of 1/0.98, with

a 98.6% assay.

EXAMPLE 4

Benzyloxycarbonyl-α-L-aspartyl-L-phenylalanine methyl ester (V, wherein R = $CH_3$ and X = $-CO-O-CH_2-C_6H_5$)

To 42.7 g of benzyloxycarbonyl-α-L-asparaginyl-L-phenylalanine methyl ester (prepared according to Examples 1a) or 2) suspended in 300 ml of dimethylformamide, 30 ml of a 6M HCl solution in dioxane was added, under stirring and cooling with water.

Thereafter, 21 g of n-butyl-nitrite,freshly distilled, was added to the reaction mixture, during about 20 minutes, under strong stirring at room temperature. The reaction was then allowed to proceed for 6-8 hours, chromatographically controlling the completion of the amidic moiety hydrolysis. The clear, slightly yellow solution was concentrated under vacuum and then diluted with 600 ml of water.

The precipitated product was recovered by filtration, washed with water and dried under vacuum, obtaining 38.5 g, 90% yield.

$[\alpha]_D^{20}$ -16.5° (c = 1, methanol).

EXAMPLE 5

a) N-Formyl-α-L-asparaginyl-L-phenylalanine methyl ester (IV, wherein R = $CH_3$ and X = HCO-)

41 Grams (0.4 moles) of triethylamine were added to a solution of 43 g (0.2 moles) of L-phenylalanine methyl ester hydrochloride, in 200 ml of dimethylformamide; the mixture was cooled to 5-10°C and a solution obtained by treating 32 g of N-formyl-L-asparagine in 150 ml of acetonitrile with 25 g of pivaloylchloride was added dropwise thereto under stirring; the mixture was left for 35-40°C for 12 hours. When the addition was over, the mixture was heated to 50-60°C for three hours, then poured in 2 kg of crushed ice and the precipitate was filtered, which, after washing with water and drying under vacuum, turned out to be sufficiently pure for the subsequent reactions. Yield 92%. M.p. 132-135°C (from methyl acetate).

Elemental analysis:

for $C_{15}H_{19}N_3O_5$ (M.W. = 321.34)

Calcd. % C = 56.07; H = 5.96; N = 13.07

Found % C = 56.22; H = 6.09; N = 13.09.

b) α-L-Aspartyl-L-phenylalanine methyl ester (aspartame) (I, wherein R = $CH_3$)

32.14 Grams of the derivative hereinabove obtained were suspended in 80 ml of glacial acetic acid and treated at about 5°C, under strong stirring, with 12 g of nitrosylsulfuric acid suspended in 50 ml of glacial acetic acid. When the nitrogen evolution ended, the mixture was stirred at room temperature for further three hours; urea was then added in small portions, until further nitrogen evolution was not observed. 11.6 Grams of sodium chloride and 0.2 moles of conc. HCl were then added, and the mixture was left at room temperature for 24 hours. The mixture was then poured on 500 g of

ice and neutralized with $NH_4OH$. By standing at 5-10°C, 27 g of aspartame, with the same characteristics reported in the Example 1c), crystallized.

EXAMPLE 6

Benzyloxycarbonyl-α-L-asparaginyl-L-phenylalanine methyl ester (IV, wherein R = $CH_3$ and X = $-CO-O-CH_2-C_6H_5$)

A solution of 53.3 g of benzyloxycarbonyl-L-asparagine (II, wherein X = $-CO-O-CH_2-C_6H_5$ and Y = OH) and 20.2 g of triethylamine in 500 ml of acetonitrile, was treated, at -20°C, with a solution of 21.7 g of ethyl chlorocarbonate in 50 ml of dichloroethane. The mixture was stirred for 2 hours at -20°C; a solution of 65 g of L-phenylalanine methyl ester hydrochloride in 500 ml of acetonitrile/pyridine 1:1 by volume was added to the mixed anhydride solution so obtained, at about -10°C, under stirring. When the addition was over, the temperature was allowed to raise to the room temperature, always under stirring, which was continued for further 8 hours. The mixture was concentrated under vacuum to half volume, then diluted with 2 liters of wat . The precipitate was filtered and thoroughly wash with 2% HCl and then with water. After drying under vacuum at 50°C, 79.4 g of product identical to that of Example 1a), which was transformed into aspartame as described in sections b) and c) of the same example, were obtained.

EXAMPLE 7

a) Trimethylsilyl-α-L-asparaginyl-L-phenylalanine methyl ester (IV, wherein R = $CH_3$ and X = -Si-$(CH_3)_3$)

L-Asparagine was subjected to trimethylsilylation, in the presence of triethylamine and in dioxane. 40.8 Grams (0.2 moles) of the raw product so obtained were treated, in 200 ml of acetonitrile, with 24 g (0.22 moles) of $SOCl_2$, at room temperature for 3 hours, then for half an hour at 50°C. The solution obtained was reacted with a solution of 65 g (0.2 moles) of L-phenylalanine methyl ester hydrochloride in 800 ml of acetonitrile/pyridine 1:1 by volume. After 10 hours at room temperature the mixture was concentrated to 2/3 of the starting volume and poured in 2 kg of crushed ice. The precipitate was filtered, thorougly washed with water and dried under vacuum at 40°C.

b) α-L-Aspartyl-L-phenylalanine methyl ester (aspartame)

The product obtained in the previous reaction was dissolved in 250 ml of anhydrous acetic acid and treated, at about 0°C, with 25.4 g of nitrosylsulfuric acid. When the nitrogen evolution ceased, the mixture was stirred for 4 hours at room temperature, some grams of urea was then added and, after further stirring for half an hour, the mixture was poured in 500 g of crushed ice (in this step the removal of the protective group from the amino group occurred). The mixture was neutralized with ammonium

hydroxide, keeping the temperature at about 5-10°C, and left to stand for 12 hours at the same temperature. 48.85 Grams of aspartame, substantially identical to that obtained in the previous examples, were obtained.

EXAMPLE 8

a) α-Bromopropionyl-L-asparaginyl-L-phenylalanine methyl ester (IV, wherein R = $CH_3$ and X = -CO-CHBr-$CH_3$)

26.7 Grams (0.1 moles) of finely pulverized α-bromopropionyl-L-asparagine (obtained from L-asparagine and α-bromopropionyl chloride according to Schotten-Baumann) were dissolved in 100 ml of dimethylformamide, by addition of 8 ml of pyridine and 28 ml of N-ethyl-piperidine. The solution, under strong stirring, was rapidly cooled to -30°C; the resulting suspension was treated with 12 g of pivaloyl chloride dissolved in 20 ml of dimethylformamide. The clear solution obtained, was treated, after about 30 minutes, with 22 g of L-phenylalanine methyl ester hydrochloride. The mixture was kept at -30°C for 30 minutes and then allowed to raise spontaneously to room temperature in about 2 hours. The solution concentrated under vacuum was poured in 1 liter of water and the suspension kept under stirring for some hours. The precipitated product was filtered, washed with water and dried under vacuum, obtaining 30 g, yield 70%.

After acid hydrolysis of the product in closed vial for 22 hours in HCl 6M at 110°, the analysis

in aminoacids gave a phenylalanine:aspartic acid ratio = 1:0.97 with a 99.2% yield.

b) α-L-Aspartyl-L-phenylalanine methyl ester (I, wherein R = $CH_3$)

21.4 Grams (0.05 moles) of α-bromopropionyl-L-asparaginyl-L-phenylalanine methyl ester, dissolved in 150 ml of dimethylformamide, were treated with 15 ml of 6M HCl solution in dioxane and then with 10.5 g of n-butyl-nitrite, added in about 20 minutes. After 6-8 hours at room temperature under constant stirring, the mixture was concentrated to dryness and the residue taken up with 100 ml of absolute methanol. 3.8 Grams (0.05 moles) of thiourea were added to the resulting mixture, and it was then refluxed for 20 minutes. The methanol suspension of the isothiouronium bromide so obtained (VI, wherein R = R' = $CH_3$ and Hal = bromine) was concentrated to dryness and the residue taken up with 100 ml of water; the pH of the solution was brought to 7-8 with $NaHCO_3$, the mixture was then heated to 40° for 2 hours. After cooling at room temperature, the mixture was acidified to pH 4.5 with 2M HCl and then concentrated under vacuum. 11.47 Grams of aspartame, substantially identical to that obtained in the previous examples, were obtained.

<u>CLAIMS for the Contracting States:</u>

AT, BE, CH, DE, FR, GB, IT, LI, LU, NL, SE

1. Process for the preparation of alkyl esters of α-L-aspartyl-L-phenylalanine having general formula (I)

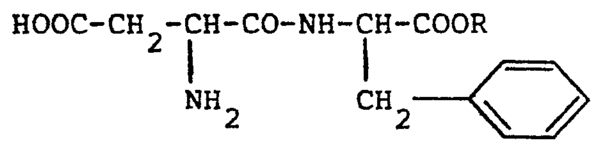

(I)

wherein R is a $C_1$-$C_5$ alkyl residue, characterized in that a L-asparagine derivative of formula (II), having the amino moiety protected, is condensated with a L-phenylalanine alkyl ester of formula (III), and that, in the dipeptide (IV) so obtained, the amido group is converted into carboxylic group by treatment with nitrite ions in acid medium, the final deprotection of the amino group taking optionally place with or without recovery of the intermediate (V) so obtained according to the scheme hereinbelow reported:

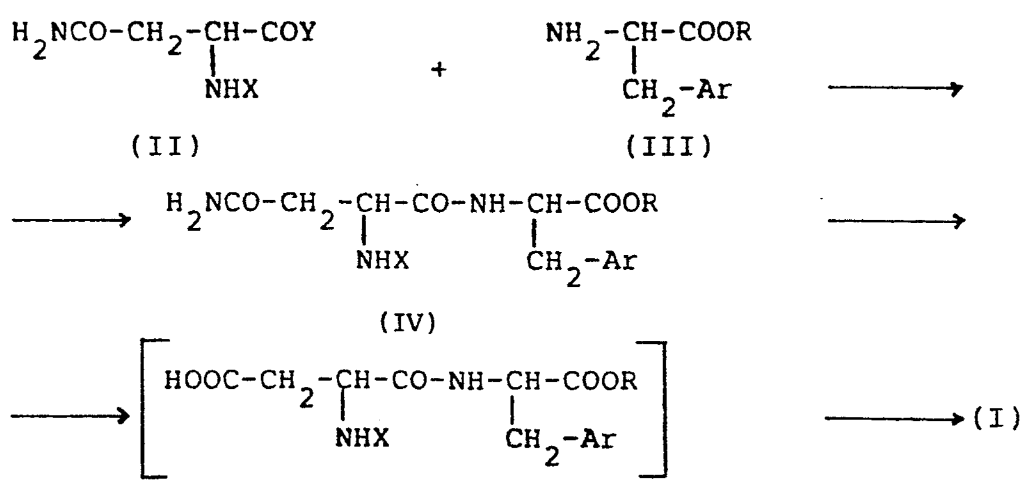

(II) (III)

(IV)

(V)

wherein:

X represents a protective group of the amino moiety;
Y represents OH or an activating atom or group;
R has the above defined meaning;
Ar represents the phenyl residue.

2. Process according to claim 1, characterized in that X is selected in the group consisting of α-haloacyl, formyl, benzyloxycarbonyl, trimethylsilyl.

3. Process according to claim 2, characterized in that X is a $-CO-\underset{Hal}{\underset{|}{CH}}-R'$ residue, wherein Hal represents chlorine or bromine, while R' is hydrogen or a linear or branched $C_1$-$C_5$ alkyl group.

4. Process according to claim 1, characterized in that the activating group Y is an alcoxycarbonyloxy, o-nitrophenoxy, o-N-succinimido residue.

5. Process according to claims 1-4, characterized in that nitrosylsulfuric acid is used for the treatment with nitrite ions in acid medium.

6. Process according to claims 1-4, characterized in that alkali metal nitrites or alkyl nitrites in the presence of mineral acids are used for the treatment with nitrite ions in acid medium.

7. Process according to claims 1-6, characterized in that for Y = OH the condensation of (II) with (III) is carried out by using dicyclohexylcarbodiimide in the presence of N-hydroxysuccinimide, in a reaction medium consisting of dimethylformamide.

8. Process according to claims 1-6, characterized in that for Y = o-nitrophenoxy, the condensation of

(II) with (III) is carried out without using other reagents, in a medium consisting of acetonitrile and pyridine.

9. Process according to claims 1-6, characterized in that for Y = OH the condensation of (II) with (III) is carried out in the presence of pivaloyl chloride.

10. Process according to the preceding claims, characterized in that, for X = α-halogenoacyl, the elimination of X from the intermediate (V) takes place by treatment with thiourea and direct hydrolysis of the isothiouronium salt so formed, at pH 7-8 and at temperatures ranging from 20 to 60°C, according to the scheme hereinbelow reported:

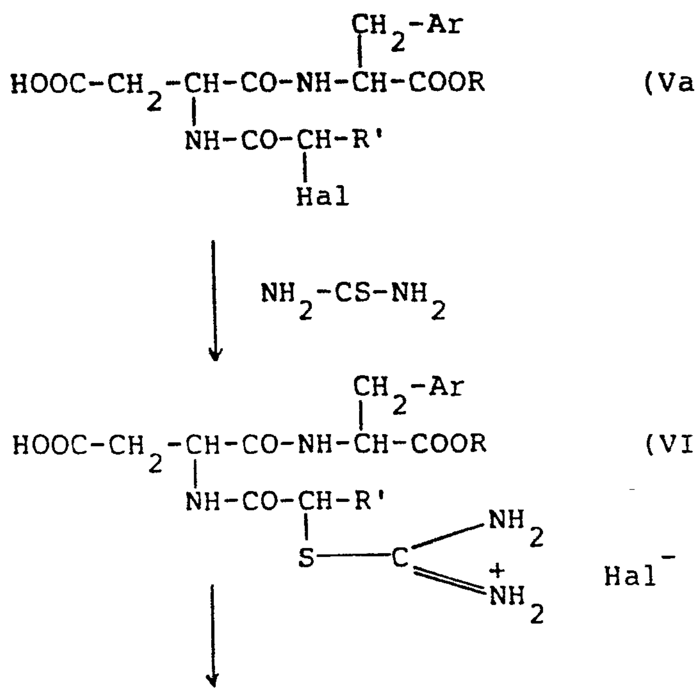


(Va)

(VI)

(I)

wherein R, Ar, Hal and R' have the above defined meanings.